# EUROPEAN PATENT APPLICATION

(11) **EP 4 198 996 A1**
(43) Date of publication of application: **21.06.2023**
(21) Application number: 21856288.2
(22) Date of filing: 13.08.2021
(51) Int. Cl.: G16H 20/70, G16H 20/30, G16H 50/20, G16H 50/30, A61B 5/16, A61B 5/11, G06F 3/01

(54) **MOVEMENT CODE-BASED EMOTIONAL BEHAVIOR ANALYSIS SYSTEM**

(30) Priority: 13.08.2020 KR 20200101769; 06.08.2021 KR 20210103895
(71) Applicant: Kim, Hyungsook, Seoul 06586 (KR)
(72) Inventor: Kim, Hyungsook, Seoul 06586 (KR)
(74) Representative: Staeger & Sperling Partnerschaftsgesellschaft mbB
(86) International application number: PCT/KR2021/010803
(87) International publication number: WO 2022/035282

(57) **Abstract**

A movement code-based emotional behavior analysis system is provided. The movement code-based emotional behavior analysis system may include an input unit that recognizes a movement of a subject, a memory that stores an image and a movement code obtained by recognizing the movement of the subject, an output unit that outputs content, and a processor that analyzes the recognized movement of the subject based on emotional behavior analysis, classifies the analyzed movement as a standardized movement code and stores the standardized movement code, and generates a movement code corresponding to an opposite behavior to be usable for correcting an emotional behavior abnormality of the subject by using the stored movement code to control the output unit to output content for inducing the opposite behavior.

## Description

### TECHNICAL FIELD

The present invention relates to a movement code-based emotional behavior analysis system, and more specifically, an emotional behavior analysis system capable of implementing a virtual environment that allows emotional behavioral characteristics to be constructed into movement codes, the behavior of a subject to be automatically recognized as a movement code using artificial intelligence based on the constructed movement code data, and behavior instruction corresponding to an analysis result to be trained based on the recognized movement code.

### BACKGROUND ART

Movement behavior is being used in the fields of cultural industry, psychiatry, and brain neuroscience as a medium of non-verbal communication to understand humans. In particular, research and development related to movement behavior is being mainly conducted in the entertainment industry (movie, animation) among the cultural industries. That is, research and development related to motion capture-based special video production has been mainly conducted. On the other hand, the understanding of human emotions and the analysis of emotions through movement have not been systematically performed.

For example, when children's distraction behavior is examined, the examination mainly includes questionnaires, interviews with parents, blood tests, brain function tests, or the like, and analysis through movement, which is a means of non-verbal communication, is not made. Even in the case of observing the movement, a method is performed in which a professional counselor continuously observes the child's behavior, which may result in a problem that the movement characteristics are not figured out in a standardized way and the abnormal behavior criteria are not accurately applied.

### DISCLOSURE OF THE INVENTION

### TECHNICAL PROBLEM

The present invention has been made in consideration of the above-mentioned problems, and an object thereof is to provide a movement code-based emotional behavior analysis system capable of recognizing a movement of a subject, classifying the recognized movement as a standardized movement code, constructing a database of emotional behavioral characteristics, and providing content that is available for correcting abnormal behaviors using the constructed database, and capable of automating movement code construction using artificial intelligence.

### TECHNICAL SOLUTION

In order to solve the aforementioned problem, according to an embodiment of the present invention, there is provided a movement code-based emotional behavior analysis system including an input unit that recognizes a movement of a subject, a memory that stores an image and a movement code obtained by recognizing the movement of the subject, an output unit that outputs content, and a processor that analyzes the recognized movement of the subject based on emotional behavior analysis, classifies the analyzed movement as a standardized movement code and stores the standardized movement code, generates a movement code corresponding to an opposite behavior to be usable for correcting an emotional behavior abnormality of the subject by using the stored movement code, and controls the output unit to output content for inducing the opposite behavior based on the generated movement code corresponding to the opposite behavior.

Further, the processor may control the output unit to output a stimulus signal for inducing the movement of the subject that is to be classified and stored as the movement code.

In addition, the processor may determine the stimulus signal for inducing the movement of the subject based on emotional behavioral characteristic test content.

Further, the processor may generate a learning model for encoding a movement code stream and generating an emotional behavioral characteristic score by using the image the movement code stored in the memory as initial training data, and may generate a movement code of the subject additionally recognized through the input unit by using the generated learning model and additionally store the generated movement code, and update the generated learning model by using the additionally stored movement code.

### ADVANTAGEOUS EFFECTS

According to various embodiments of the present invention as described above, it is possible to develop an artificial intelligence learning model that understands human behavior, particularly non-verbal communication, by securing standardized movement codes. In addition, it is possible to analyze emotional behavior through the movement codes and provide content that is available for correcting emotional behavior abnormalities, thereby producing an effect of providing a solution to solve mental health problems. In addition, by constructing movement codes, it is possible to acquire behavioral data related to unresolved health problems at each stage of the entire life, enabling early prediction and management of diseases.

### BRIEF DESCRIPTION OF THE DRAWINGS

FG. 1 is a block diagram for describing a configuration of a movement code-based emotional behavior analysis system according to an embodiment of the present invention;
FIG. 2 is a diagram showing an example of extracting behaviors related to emotional behavioral characteristics in a movement code construction device according to an embodiment of the present invention;
FIG. 3 is a block diagram for describing a configuration of a movement code construction device according to an embodiment of the present invention;
FIG. 4 is a diagram showing an example of encoding a movement code by applying sensor fusion technology according to an embodiment of the present invention;
FIG. 5 is a diagram showing movement codes according to various embodiments of the present invention;
FIG. 6 is a diagram showing a configuration of movement data according to an embodiment of the present invention;
FIG. 7 is a diagram showing an example of extracting behavioral characteristics through behavioral stimuli according to an embodiment of the present invention;
FIG. 8 is a diagram showing a scenario in which content is created based on adolescent personality and mental health problems screening questionnaire (AMPQ)-III according to an embodiment of the present invention;
FIG. 9 is a diagram showing an example of extracting an opposite behavior according to an embodiment of the present invention;
FIG. 10 is a diagram showing an example of constructing a standard movement code applicable to AMPQ-III according to an embodiment of the present invention;
FIG. 11 is a diagram showing an example of determining educational content by applying a movement code corresponding to an opposite behavior according to an embodiment of the present invention;
FIG. 12 is a diagram for describing an operation of a movement code automation device according to an embodiment of the present invention;
FIGS. 13 and 14 are conceptual diagrams of a movement code-based emotional behavior analysis system according to various embodiments of the present invention;
FIG. 15 is a diagram illustrating a movement code-based behavior test and an educational content user scenario according to an embodiment of the present invention;
FIG. 16 is a diagram showing a process of constructing emotional behavioral data into movement codes according to an embodiment of the present invention;
FIG. 17 is a schematic diagram showing a movement code automation system according to an embodiment of the present invention;
FIG. 18 is a schematic diagram illustrating a movement code-based scoring system according to an embodiment of the present invention; and
FIG. 19 is a schematic diagram illustrating a movement code-based scoring system according to an embodiment of the present invention in more detail.

### MODE FOR CARRYING OUT THE INVENTION

Hereinafter, various embodiments disclosed herein will be described with reference to the accompanying drawings. However, this is not intended to limit the technology described in the present disclosure to specific embodiments, and it is to be construed to include various modifications, equivalents, and/or alternatives of embodiments of the present disclosure. With regard to the description of the drawings, similar reference numerals may be used to refer to similar elements.

As used herein, terms and phrases such as "have", "may have", "include", or "may include" indicate the existence of features (e.g., numbers, functions, actions, or parts such as components), and do not exclude the existence of additional features.

As used herein, the phrases "A or B", "at least one of A and/or B", or "one or more of A and/or B" may include all possible combinations of the items listed together. For example, "A or B", "at least one of A and B", or "at least one of A or B" may indicate all of (1) including at least one A, (2) including at least one B, or (3) including both at least one A and at least one B. As used herein, the terms such as "first", "second", "the first", or "the second" may modify various components, regardless of order and/or importance, and are merely used to distinguish one component from another, but does not limit the components.

As used herein, the phrase, "configured to (or set to)", may be interchangeably used with, for example, "suitable for", "having the capacity to", "designed to", "adapted to", "made to", or "capable of', depending on the circumstances. The phrase "configured (or set) to" may not necessarily mean only "specifically designed to" in hardware. In some circumstances, the phrase "device configured to" may mean that the device "can" perform an operation with other devices or parts. For example, the phrase "processor configured (or set) to perform A, B, and C" may mean a dedicated processor (e.g., embedded processor) for performing corresponding operations, or a generic-purpose processor (e.g., CPU or application processor) that performs the operations by executing one or more software programs stored in a memory device.

Hereinafter, the present invention will be described in detail with reference to the drawings.

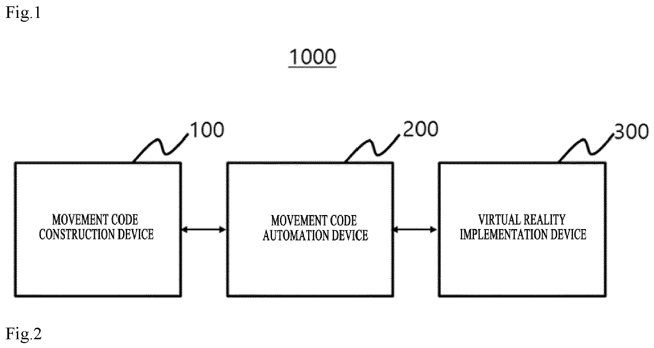

FIG. 1 is a block diagram showing elements constituting a movement code-based emotional behavior analysis system 1000 according to an embodiment of the present invention. Referring to FIG. 1, the emotional behavior analysis system 1000 may include a movement code construction device 100, a movement code automation device 200, and a virtual environment implementation device 300. The division of devices is for convenience of description and is not limited to the emotional behavior analysis system 1000 having to operate by three devices. For example, it goes without saying that the function of the movement code construction device 100 and the function of the movement code automation device 200 may be performed in one electronic device.

The movement code construction device 100 may recognize the movement of a subject (e.g., behavior, gaze, facial expression, voice, or the like) based on multi-modal sensing, and may classify and analyze the recognized movement according to characteristics. Further, the movement code construction device 100 may construct a standardized movement code capable of expressing not only the form but also the quality of the recognized movement. The movement code may include indicators that objectively describe movements expressed externally due to the intention of the subject. For example, the movement code may include indicators representing weight, time, space, and flow.

Therefore, when the movement code is used, a determination may be objectively made as to whether the behavior is light or strong, whether the movement is sudden or sustained, whether the movement is a direct gazing movement or indirect gazing movement, or whether the movement is free-flowing or bound.

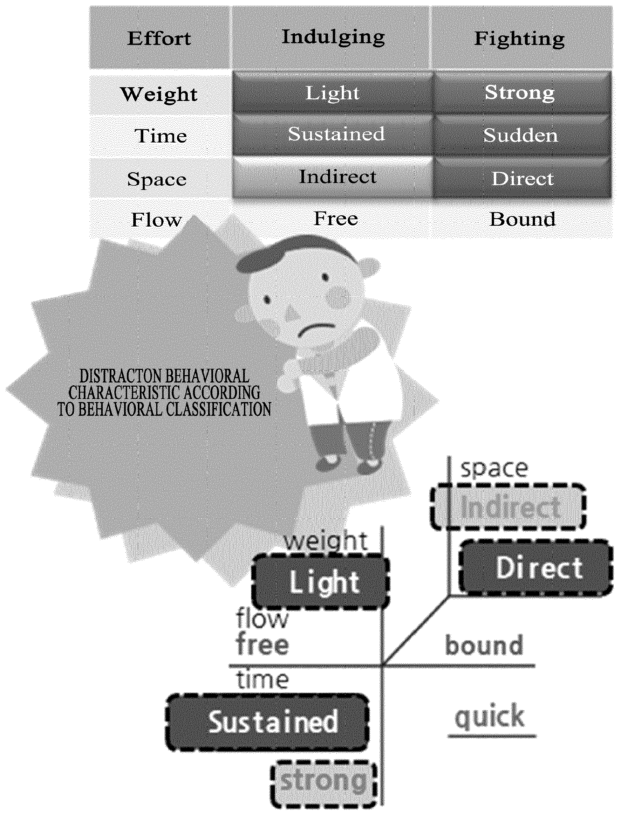

The movement code construction device 100 may construct all movements of daily life into movement codes, but may also construct movement codes with a specific goal. For example, the movement code construction device 100 may extract behaviors related to emotional behavioral characteristics. As in the embodiment of FIG. 2, when the subject cannot sit still or continues to move the limbs, the movement code construction device 100 may extract behavior elements of "strong", "sustained", and "indirect".

The movement code construction device 100 may store, in a database, not only the constructed movement code but also image data captured to recognize the subject. For example, by matching and storing an image and a movement code in the movement code construction device 100, the stored database may be used as training data necessary for training an artificial intelligence learning model for automating movement code construction.

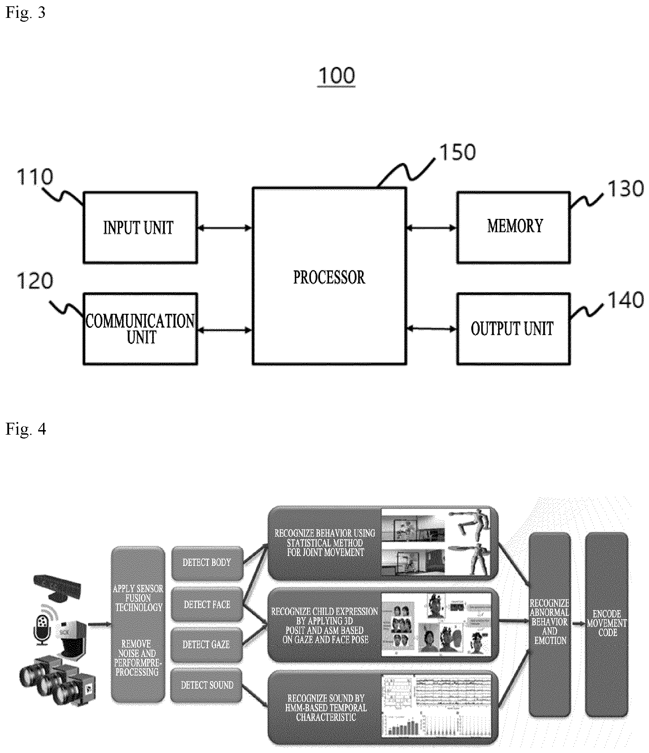

The movement code construction device 100 may include the same components as the embodiment of FIG. 3. The movement code construction device 100 may include an input unit 110, a communication unit 120, a memory 130, an output unit 140, and a processor 150. However, not all of the above-described components are necessarily required when a method for constructing a movement code according to an embodiment of the present invention, and various components may be additionally included in addition to the above-described components.

The input unit 110 may sense the movement of the subject using various sensor devices. The input unit 110 may be constituted by various devices, such as an image sensor, motion sensor, camera, charge coupled device (CCD), complementary metal oxide semiconductor (CMOS) device, Kinect, and microphone, to detect the body, face, gaze, sound, and movement of the subject.

The communication unit 120 may communicate with an external device such as a server using a wired or wireless network. For example, the communication unit 120 may transmit the constructed movement code to the movement code automation device 200 and the virtual reality implementation device 300. The communication unit 120 may use various methods such as Bluetooth, Zigbee communication, WiFi, infrared (IR) communication, and Near Field Communication (NFC) as a wireless communication method. In addition, the communication unit 120 may use a high-definition multimedia interface (HDMI), low voltage differential signaling (LVDS), a local area network (LAN), or the like, as a wired communication method.

The memory 130 may store various modules, software, and data for driving the movement code construction device 100. For example, the memory 130 may store software for analyzing the image sensed by the image sensor, software for converting an image analysis result into a movement code, the constructed movement code data, data acquired at the input unit 110 (image, audio, and the like), control signal data such as a communication protocol with an external device, or the like.

The memory 130 may have a form of a flash memory, a hard disk drive (HDD), or a solid state drive (SSD). For example, the memory 130 may include a read-only memory (ROM) for storing programs and/or applications for performing operations of the movement code construction device 100, and a random-access memory (RAM) for temporarily storing data with the operation of the movement code construction device 100. In addition, the memory 130 may further include an electrically erasable and programmable ROM (EEPROM) for storing various reference data.

The output unit 140 may output a stimulus signal for inducing a movement of the subject, content that is available in naturally inducing a movement of the subject, and the like. The output unit 140 may be provided in various forms, such as a display for outputting images, a speaker for outputting audio, a light emitting device (LED) light source for outputting a flickering signal, and a virtual reality (including virtual reality (VR), augmented reality (AR), and mixed reality (MR)) output device.

The processor 150 may control the above-described components of the movement code construction device 100. For example, the processor 150 may control the communication unit 120 to transmit constructed movement code data to an external device. In addition, the processor 150 may control the output unit 140 to output content or the stimulus signal for inducing a movement of the subject for analyzing emotional behavioral characteristics. The processor 150 may be manufactured in the form of one or a plurality of hardware chips and mounted in the movement code construction device 100. For example, the processor 150 may be manufactured as a general-purpose processor (e.g., CPU or application processor) or may be manufactured as a dedicated hardware chip for artificial intelligence.

The processor 150 may integrate or pre-process data received from at least one of the devices constituting the input unit 110. For example, as in the embodiment of FIG. 4, the processor 150 may integrate movement images of the subject sensed by a camera, an image sensor, an infrared sensor, a UWB sensor, a radar sensor, and the like, by applying sensor fusion technology. The processor 150 may perform a preprocessing process of time-sequentially synchronizing pieces of data measured by various sensors. Further, the processor 150 may perform preprocessing, such as noise removal, on data received through the input unit 110.

In addition, the processor 150 may recognize a behavior and an emotion using the preprocessed data. For example, the processor 150 may recognize the behavior of the subject by analyzing movements of joints of the body detected by an image sensor or the like. The processor 150 may use a statistical method to analyze joint movement. In addition, the processor 150 may recognize the facial expression of the subject through the face and gaze direction detected by the image sensor or the like. The processor 150 may use three-dimensional pose for orthography, scaling with iterations (POSIT) and/or an active shape model (ASM), or the like to recognize the expression. Further, the processor 150 may recognize the voice of the subject detected by the microphone or the like. The processor 150 may increase recognition accuracy by detecting a voice section including the speech of the subject and a voice section not including the speech of the subject from a voice signal. The processor 150 may use a hidden Markov model (HMM)-based temporal characteristic method to recognize the voice of the subject.

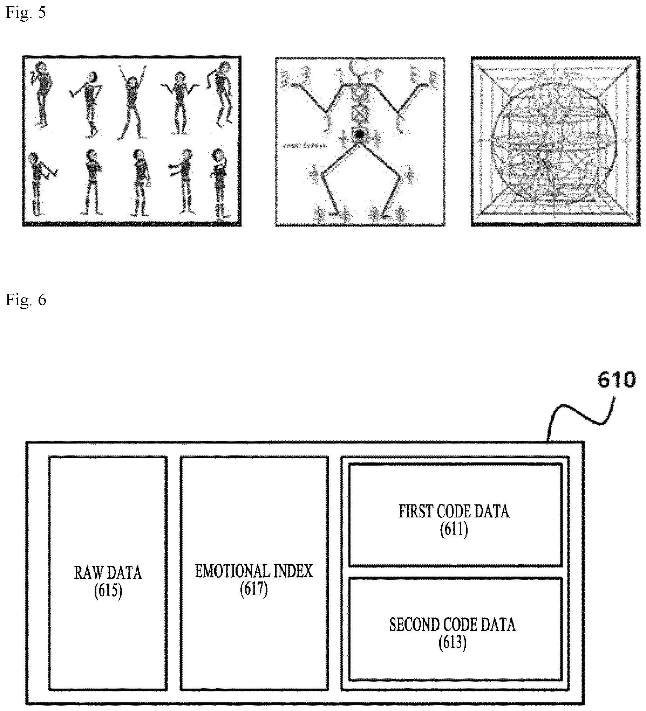

In this way, the processor 150 may collect various sensing information to recognize the behavior and/or emotion of the subject. The processor 150 may analyze and classify the recognized behavior and construct the classified behavior into a movement code. For example, the processor 150 may classify the recognized subject's behavior based on emotional behavior analysis. In addition, by assigning a standardized movement code for each classified behavior, the processor 150 may construct a movement code standard database. The movement code database may additionally store not only the movement code but also a behavior image of the subject determined by the corresponding movement code. The movement code refers to a recording and coding of human gestures and behaviors like a labanotation (recording dance movements with certain symbols or pictures like musical scores). FIG. 5 is a diagram showing exemplary movement codes. As shown in FIG. 5, the movement code may be built in a form obtained by mimicking human motion, or may be built as structured data having a preset length coded in numbers or letters. The movement code constructed in this way is highly utilized as data for artificial intelligence learning in that it has a standardized structure.

For example, the constructed movement code data 610 may have a form as shown in FIG. 6. Referring to FIG. 6, movement code data 610 may include movement codes 611 and 613 and raw data 615 such as movement, gesture, face, facial expression, and voice. In addition, the movement code data 610 may further include an emotional index 617 determined from the subject's behavior, expression, voice, and the like. The emotional index 617 may be used as reference data to determine the subject's emotional abnormality. For example, when different subjects have different emotional indices even when they have the same movement code, opposite behaviors to be provided to the respective subjects may be differently determined.

In addition, the movement codes 611 and 613 included in the movement code data 610 may include at least one of two types of code data. The first code data 611 may be data obtained by attaching an annotation to ground truth data, and the second code data 613 may be data given through a movement code recognition model to be described later. For example, in data for training the movement code recognition model in the beginning, there will be a plurality of movement codes mainly containing only the first code data 611, and after the movement code recognition model that has been trained is used, there will be a plurality of movement codes mainly containing only the second code data 613. The two types of code data may be applied complementary to each other.

As described above, the movement code construction device 100 may recognize the movement of the subject and construct the recognized movement into a standardized movement code. In addition to passively recognizing the movement of the subject, the movement code construction device 100 may induce the movement of the subject to be analyzed by providing a stimulus to the subject. For example, the processor 150 may control the output unit 140 to provide a visual stimulus "A" to the subject. The subject receiving the visual stimulus may take different behaviors with individual emotional behavioral characteristics. The movement code construction device 100 may recognize the movement of a subject who has taken a behavioral response of "B" and classify and store it as a movement code corresponding to autism, and may recognize the movement of a subject who has taken a behavioral response of "C" and classify and store it as a movement code corresponding to no emotional abnormality. That is, the movement code construction device 100 may induce motions required for movement analysis with a specific purpose, such as emotional behavior analysis, and may construct the movement code by analyzing the induced behavioral response.

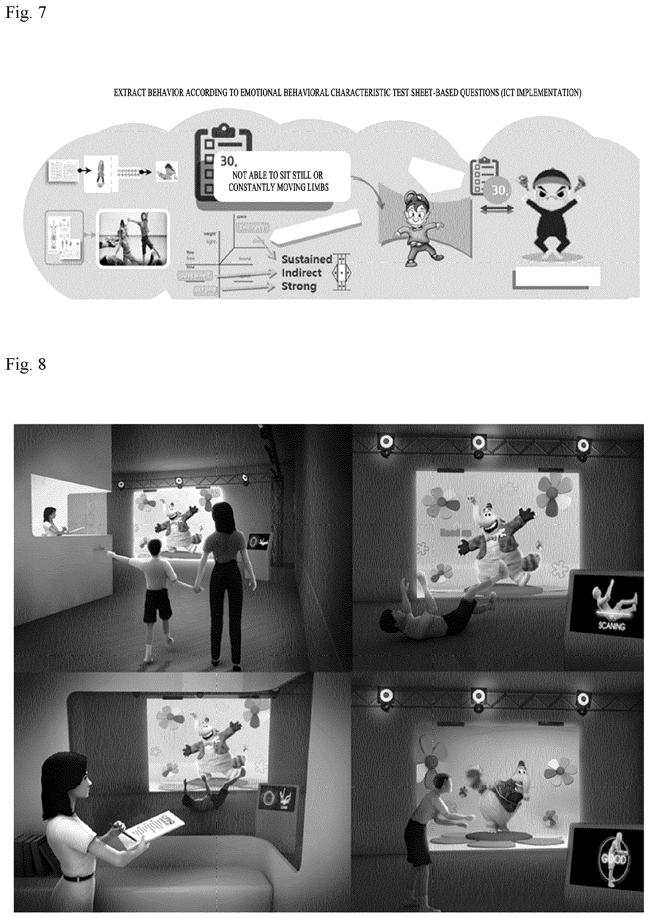

Referring to FIG. 7, the movement code construction device 100 may provide a behavioral stimulus for inducing a required movement to the subject to extract behavioral characteristics required to test emotional behavioral characteristics. In the case of receiving all of the daily life of the subject as it is, the amount of data is large, but there may be a problem that the high quality data required to extract the behavioral characteristics may be small. Therefore, the present invention has been made to be available to collect high-quality data by actively inducing the behavior of the subject. For example, when the emotional behavioral characteristic to be tested is "characteristic of not being able to sit still or constantly moving the limbs", the movement code construction device 100 may provide the subject with a behavioral stimulus for inducing the corresponding behavior. In addition, the movement code construction device 100 may extract a behavioral characteristic according to the behavioral response of the subject, and construct the extracted behavioral characteristic into a movement code.

For stimuli provided by the movement code construction device 100 for inducing the movement of the subject, various stimuli such as visual, auditory, and olfactory stimuli may be provided. In addition, through content such as virtual reality and games that include behavior-inducing stimuli, the movement code construction device 100 may induce the movement required for analysis and construct the movement into a movement code while the subject is enjoying the content without recognition. The operation of the movement code construction device 100 may be provided through the virtual reality implementation device 300.

The movement to be induced by the movement code construction device 100 may be determined based on the emotional behavioral characteristic test content. The test content may contain details in which emotional behavior disorder, stress, mild cognitive impairment, early dementia, ADHD, and the like, may be determined through non-verbal communication means (movement or the like). For example, using Diagnostic & Statistical Manual of Mental Disorders 5^{th} Ed (DSM-V), Mini-Mental State Examination for Dementia Screening (MMSE-DS), Adolescent Mental Problem Questionnaire-III (AMPQ-III), or the like, emotional behavioral characteristic test content may be generated. FIG. 8 is a diagram showing a scenario in which content is created based on AMPQ-III. In the embodiment of FIG. 8, the movement code construction device 100 may provide content using animation characters to induce a movement of raising a hand while lying down, bending a body, and so on.

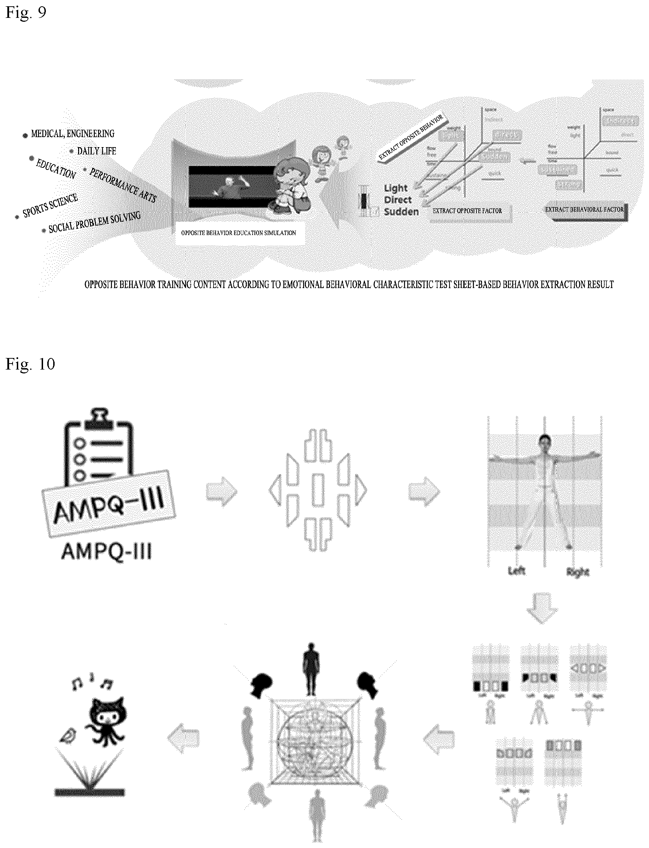

The movement code construction device 100 may extract an opposite element that is available for inducing an opposite behavior by extracting a behavior element stored as a movement code. For example, in FIG. 9, when the subject may not sit still or continues to move the limbs, the movement code construction device 100 may store the behavior of the subject as the movement code having behavior elements of "strong", "sustained", and "indirect". Based on the stored movement code, the movement code construction device 100 may determine that a corrective behavior (opposite action) for a distracted behavior has behavior elements of "light", "sudden", and "direct".

Further, the movement code construction device 100 may correct the emotional abnormality of the subject by providing educational content that is available for inducing the opposite behavior. For example, the movement code construction device 100 may correct the emotional abnormality by inducing the opposite behavior by issuing a behavioral instruction to the subject using motion graphics or language guides. For another example, the movement code construction device 100 may correct emotional abnormality by naturally inducing the subject to perform the opposite behavior in a state in which the subject is not recognized in the content. The operation of the movement code construction device 100 may be provided through the virtual reality implementation device 300.

That is, the movement code construction device 100 may determine content to be provided based on the constructed movement code. Further, since motions corresponding to a specific movement code are determined, it is effective to use the movement code to generate content to be provided.

As described above, the movement code construction device 100 may need to induce additional movement for the emotional behavioral characteristic test, or may need to induce the opposite behavior for correcting the emotional behavior. Accordingly, the movement code construction device 100 may determine the movement code based on the multimodal data received through the input unit 110, and determine what movement to be induced through the determined movement code.

To this end, first, the movement code construction device 100 may receive input from the user about the emotional behavior to be tested and corrected. For example, the movement code construction device 100 may receive, from the user, an emotional behavior (emotional behavioral disorder, stress, mild cognitive impairment, early dementia, ADHD, or the like) to be tested and corrected. Based on the received emotional behavior to be tested and corrected, the movement code construction device 100 may determine whether the movement code determined based on the received multimodal data is sufficient data to perform a target emotional behavior test.

When the data is not sufficient to perform the target emotional behavior test, the movement code construction device 100 may determine a movement to be additionally induced for the emotional behavioral characteristic test. Further, the movement code construction device 100 may generate a movement code corresponding to the determined movement to be additionally induced, and may generate content for inducing a corresponding behavior based on the generated movement code. The generated content may be provided through the output unit 140 or the virtual environment implementation device 300, and the movement code construction device 100 may collect movements of the subject corresponding to the provided content and generate the movement code required for the test.

Conversely, when the data is sufficient to perform the target emotional behavior test, the movement code construction device 100 may generate a movement code corresponding to the opposite behavior for behavior correction. In addition, the movement code construction device 100 may generate content for inducing the opposite behavior based on the generated movement code. Likewise, the generated content may be provided through the output unit 140 or the virtual environment implementation device 300.

When a plurality of movement codes required for inducing or correcting the behavior are determined, the movement code construction device 100 may also determine a movement code corresponding to intermediate movement connecting the plurality of generated movement codes. For example, when a motion of lowering the left hand down and a motion of raising the left hand up to the right are required, movement codes in which the left hand is located on the torso, which is an intermediate path, may be determined as a candidate group. Subsequently, the movement code construction device 100 may determine that, in the candidate group, a movement code with the indicator representing weight, time, space, and flow that is most similar to the plurality of determined movement codes is the intermediate movement code. For example, a movement code that minimizes the indicator difference may be determined as an intermediate movement code. Through the intermediate movement code connecting the plurality of determined movement codes, the movement code construction device 100 may generate a movement code stream, and may generate content using the movement code stream. The movement code construction device 100 may repeat the process of determining the intermediate movement code until all movements are connected to each other.

In this way, the movement code construction device 100 may generate a movement code for producing content according to the movement code determined based on the multimodal data and the emotional behavior to be targeted. Giving further description, it should be noted that, in the movement code construction device 100, a movement code corresponding to a motion that the subject is induced to perform is first generated, and content output from the output unit 140 or the virtual reality implementation device 300 or a robot (not shown) capable of interacting with the subject is not for the motion corresponding to the generated movement code, but intended to induce the motion corresponding to the generated movement code. For example, assuming a case of inducing a downward movement with both hands, the movement code construction device 100 may generate a movement code corresponding to the downward movement with both hands. Further, the movement code construction device 100 may output a motion of a robot or a virtual reality character spreading both palms upward by using the generated movement code. This is because the robot or virtual reality character needs to output the motion of spreading both palms upward to induce the motion of lowering the two hands down, which is the motion of the subject to be targeted.

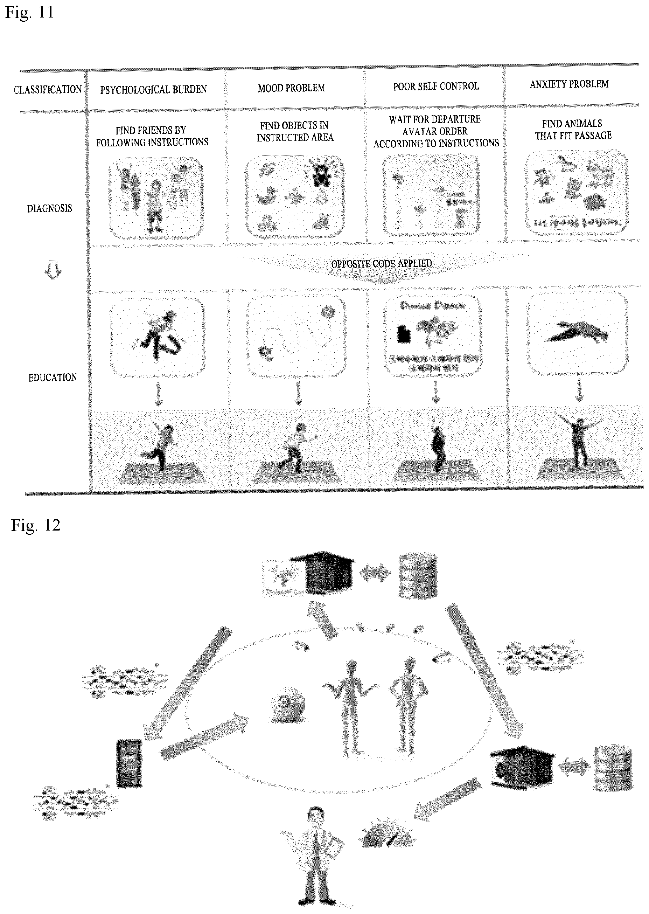

Referring to FIGS. 10 and 11, the movement code construction device 100 may construct a standard movement code applicable to an emotional behavior disorder test or education. Further, the movement code construction device 100 may test emotional behavioral characteristics or personality characteristics based on the movement code, and may determine a movement code corresponding to the opposite behavior to correct the problematic behavior. Subsequently, the movement code construction device 100 may provide educational content to which the determined movement code corresponding to the opposite behavior is applied.

The movement code automation device 200 may use artificial intelligence technology to automatically recognize movement codes. The movement code automation device 200 may perform deep learning based on multi-image and/or skeleton, or perform deep learning based on multi-modal sensing data. As the initial training data, a movement code database built in the movement code construction device 100 may be used. The movement code database may include image data obtained by photographing the movement of a subject, audio data for the subject, and the like. Further, the movement code database may include pieces of movement code information determined in association with corresponding pieces of stored image data. An example of movement code data constituting the movement code database may be as shown in FIG. 6. Through the data, the movement code automation device 200 may learn a criterion for determining a movement code corresponding to image information.

The movement code automation device 200 may include a processor for AI training and data recognition through the trained AI. Descriptions of an input unit, a communication unit, and a memory that may be included in the movement code automation device 200 are replaced with descriptions of corresponding components of the movement code construction device 100. The processor 210 may be manufactured in the form of one or a plurality of hardware chips and mounted in the movement code automation device 200. For example, at least one of a plurality of hardware chips constituting the processor may be manufactured in the form of a dedicated hardware chip for artificial intelligence, or may be manufactured as a part of an existing general-purpose processor (CPU or AP) or an IP for a specific function and mounted in the movement code automation device 200.

Referring to FIG. 12, using the movement code database, the processor may learn the criteria for encoding into movement codes through analysis of the recognized movement data, and generating emotional behavioral characteristic scores based on emotional behavioral characteristic criterion data. According to the learned criterion, the processor may classify the movement of the subject recognized from an image or sensor as one of preset movement codes. In addition, the processor may calculate an emotional behavioral characteristic score through the movement code stream recognized by the classification. The processor may determine what kind of emotional disorder the subject has through the calculated emotional behavioral characteristic score. Alternatively, the processor may not directly determine an emotional disorder, but may provide the calculated emotional behavioral characteristic score to an expert so that the expert may refer to it for diagnosis.

Further, the processor may generate a movement code stream for the opposite behavior using the recognized movement code stream. Further, the processor may generate physical actions, stimuli, language guides, or the like, capable of inducing the subject to perform the opposite behavior using the generated movement code stream for the opposite behavior.

The processor may additionally store, in the database, image data newly input in the process of determining the movement code using the generated learning model and a movement code determined corresponding to the newly input image data. Further, the processor may update the generated learning model using the additionally stored image data and movement code information.

The processor may additionally store, in the database, image data newly input in the process of determining the movement code using the generated learning model and a movement code determined corresponding to the newly input image data. Further, the processor may update the generated learning model using the additionally stored image data and movement code information.

The virtual reality implementation device 300 may provide, based on the virtual environment, content for inducing the opposite behavior for correcting the emotional behavior of the subject based on the movement code. The virtual environment referred to herein is a concept that encompasses all of virtual reality, augmented reality, and mixed reality. Taking augmented reality as an example, the virtual reality implementation device 300 may capture a general image, generate an augmented reality image by combining content that is available for inducing the opposite behavior, and provide the generated augmented reality image to the subject. In this way, the virtual reality implementation device 300 may naturally induce the subject to take the opposite behavior that is available for correcting the emotional behavior through the content. The virtual reality implementation device 300 may provide content through a display device, a projector, a VR device, a robot, and the like.

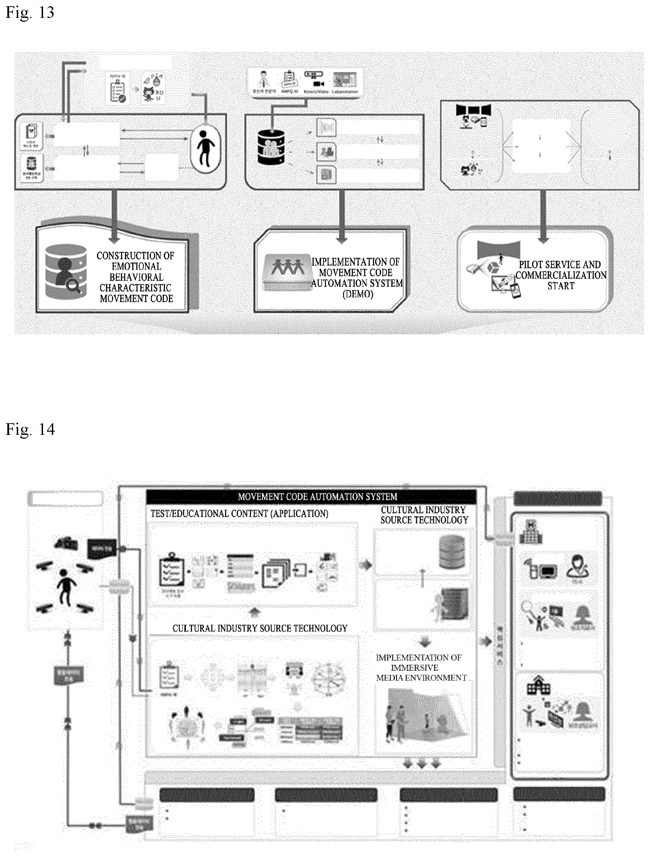

As described above, according to various embodiments, the movement code-based emotional behavior analysis system 1000 may construct movement codes related to emotional behavioral characteristics, may automatically construct movement codes using artificial intelligence technology, and may provide content (e.g., immersive content such as virtual reality) for correcting problematic emotional behavior based on the recognized movement code. FIGS. 13 and 14 are conceptual diagrams of the movement code-based emotional behavior analysis system 1000.

Hereinafter, operations of the movement code-based emotional behavior analysis system 1000 according to another embodiment of the present invention will be described with reference to additional drawings.

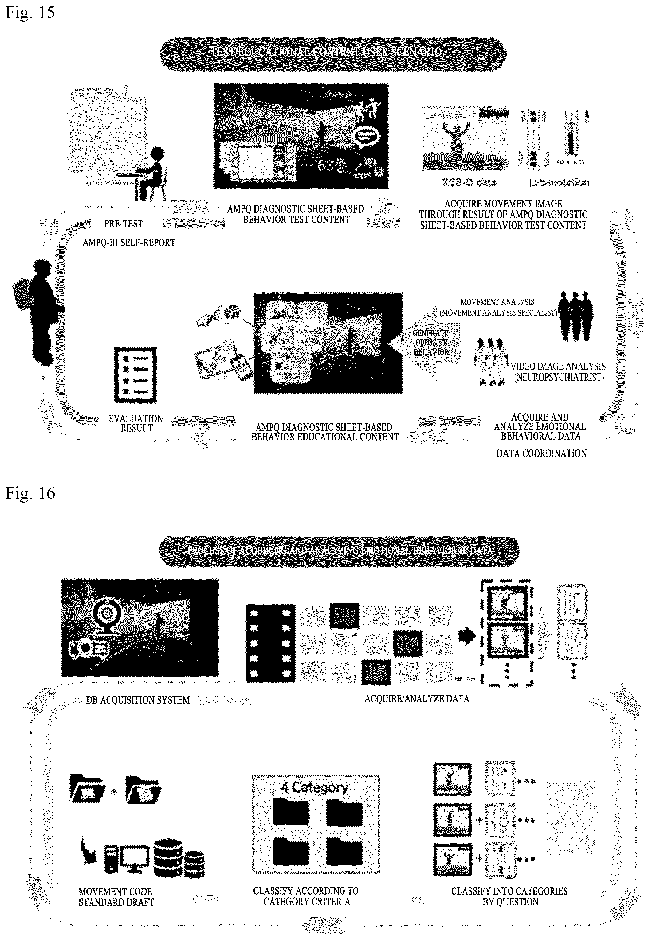

Referring to FIG. 15, the movement code-based emotional behavior analysis system 1000 may provide AMPQ diagnostic sheet-based behavior test content to a subject and acquire a movement image of the subject. Subsequently, the movement code-based emotional behavior analysis system 1000 may acquire and analyze emotional behavioral data through movement and video image analysis. By generating the opposite behavior based on the analysis, the movement code-based emotional behavior analysis system 1000 may generate AMPQ diagnosis sheet-based behavioral education content.

Describing the process of acquiring and analyzing emotional behavioral data in more detail with reference to FIG. 16, the movement code-based emotional behavior analysis system 1000 may extract required data for each question of the diagnosis sheet from an image obtained by photographing the subject's movement. In addition, the movement code-based emotional behavior analysis system 1000 may add movement code notation to the acquired data and classify it as data for each question. Subsequently, the movement code-based emotional behavior analysis system 1000 may construct a standard movement code database by making a database of the classified image data and movement code (notation) data together.

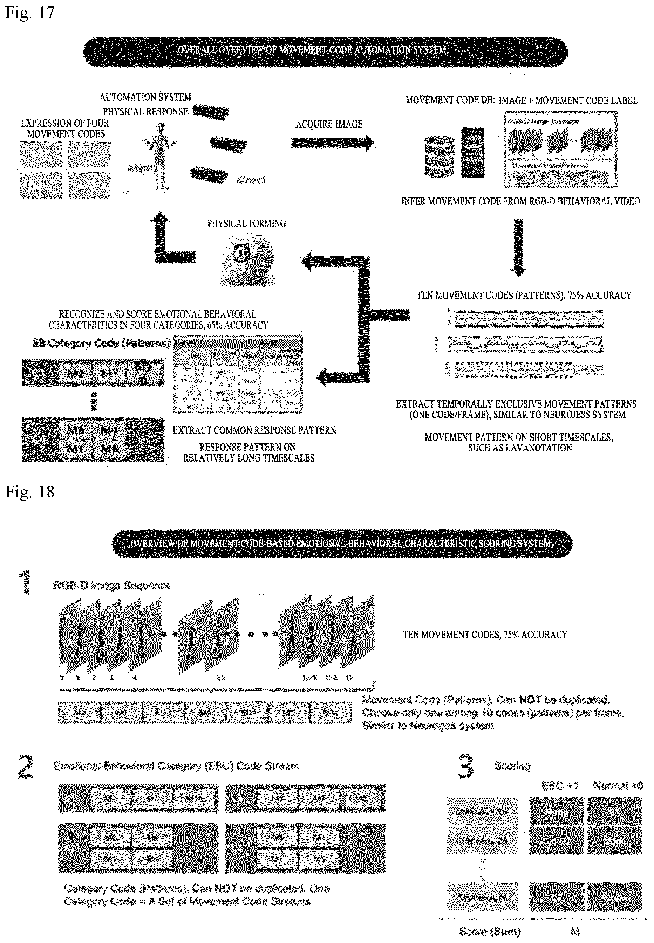

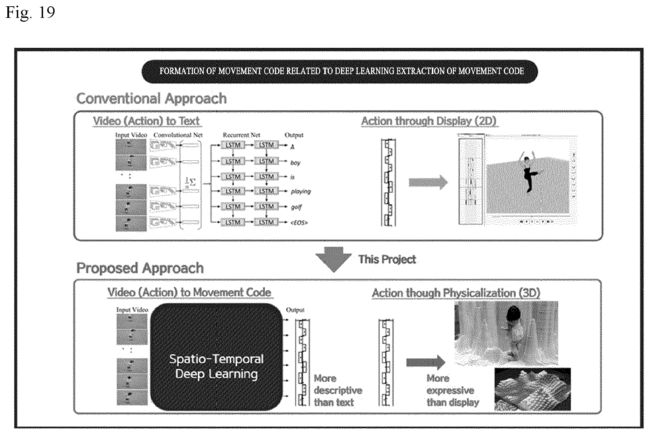

Referring to FIG. 17, the movement code-based emotional behavior analysis system 1000 may automatically recognize the movement code. The movement code-based emotional behavior analysis system 1000 may acquire a movement code by inferring the movement code from an RGB-D behavior video. Further, the movement code-based emotional behavior analysis system 1000 may generate data useful for diagnosing emotional behavior through scoring related to emotional behavioral characteristics. More specifically, referring to FIG. 18, the movement code-based emotional behavior analysis system 1000 may recognize and score four categories of emotional behavioral characteristics using 10 movement codes. Further, the movement code-based emotional behavior analysis system 1000 may extract the movement codes using a deep learning technique as shown in FIG. 19.

As described above, according to various embodiments of the present invention as described above, it is possible to develop an artificial intelligence learning model that understands human behavior, particularly non-verbal communication, by securing standardized movement codes. In addition, it is possible to analyze emotional behavior through the movement codes and provide content that is available for correcting emotional behavior abnormalities, thereby producing an effect of providing a solution to solve mental health problems. In addition, by constructing movement codes, it is possible to acquire behavioral data related to unresolved health problems at each stage of the entire life, enabling early prediction and management of diseases.

Meanwhile, the terms "unit" or "module" used herein may include units constituted by hardware, software, or firmware, and may be interchangeable with terms such as logic, logic blocks, parts, or circuits, for example. A "unit" or "module" may be an integral part or a minimal unit or part thereof that performs one or more functions. For example, the module may be constituted by an application-specific integrated circuit (ASIC).

Various embodiments of the present invention may be implemented by software including instructions stored in a storage medium (machine-readable storage medium) readable by a machine (e.g., a computer). The machine is a device capable of calling an instruction stored in a storage medium and operating according to the called instruction, and may include an electronic device (e.g., the movement code construction device 100) according to the disclosed embodiments. When the instruction is executed by a processor, the processor may perform a function corresponding to the command directly or by using other elements under the control of the processor. The instruction may include a code generated or executed by a compiler or interpreter. The machine-readable storage medium may be provided in the form of a non-transitory storage medium. Here, "non-transitory" only means that the storage medium does not contain a signal and is tangible, but does not distinguish temporary storage of data from semi-permanently storage, in the storage medium.

According to an embodiment, a method according to various embodiments disclosed herein may be included and provided in a computer program product. The computer program product may be traded as a product between a seller and a buyer. The computer program product may be distributed in the form of a machine-readable storage medium (e.g., compact disc read only memory (CD-ROM)) or online through an application store (e.g., Play Store^{™}). If distributed online, at least part of the computer program product may be temporarily generated or at least temporarily stored in the storage medium, such as memory of the manufacturer's server, a server of the application store, or a relay server.

Each component (e.g., module or program) according to various embodiments may be made up of a singular or a plurality of entities, and some of the aforementioned sub-components may be omitted, or other sub-components may be further included in various embodiments. Alternatively or additionally, some components (e.g., modules or programs) may be integrated into one entity, performing the same or similar functions performed by respective corresponding components before integration. According to various embodiments, operations performed by a module, program, or another component may be sequentially, parallelly, repeatedly, or heuristically executed, at least some operations may be executed in a different order, omitted, or other operations may be added.

## Claims

1. A movement code-based emotional behavior analysis system comprising:
an input unit that recognizes a movement of a subject;
a memory that stores an image and a movement code obtained by recognizing the movement of the subject;
an output unit that outputs content; and
a processor that analyzes the recognized movement of the subject based on emotional behavior analysis, classifies the analyzed movement as a standardized movement code and stores the standardized movement code, generates a movement code corresponding to an opposite behavior to be usable for correcting an emotional behavior abnormality of the subject by using the stored movement code, and controls the output unit to output content for inducing the opposite behavior based on the generated movement code corresponding to the opposite behavior.

2. The movement code-based emotional behavior analysis system of claim 1, wherein the processor
determines whether it is possible to generate the movement code corresponding to the opposite behavior by using the stored movement code, and
controls the output unit to output a stimulus signal for inducing a movement of the subject corresponding to an additional movement code when a determination is made that the additional movement code is required to generate the movement code corresponding to the opposite behavior.

3. The movement code-based emotional behavior analysis system of claim 2, wherein the processor determines the stimulus signal for inducing the movement of the subject based on emotional behavioral characteristic test content.

4. The movement code-based emotional behavior analysis system of claim 1, wherein the processor
generates a learning model for encoding a movement code stream and generating an emotional behavioral characteristic score by using the image the movement code stored in the memory as initial training data, and
generates a movement code of the subject additionally recognized through the input unit by using the generated learning model and additionally stores the generated movement code, and updates the generated learning model by using the additionally stored movement code.

5. The movement code-based emotional behavior analysis system of claim 1, wherein the movement code includes indicators representing weight, time, space, and flow.

6. The movement code-based emotional behavior analysis system of claim 5, wherein the processor derives an intermediate movement code candidate group of a plurality of movement codes based on the plurality of movement codes corresponding to the generated opposite behaviors, determines an intermediate movement code that minimizes an indicator difference with the plurality of movement codes in the derived candidate group, generates a movement code stream by using the plurality of movement codes and the determined intermediate movement code, and outputs content for inducing the opposite behavior by using the generated movement code stream.
